# DEMANDE DE BREVET EUROPEEN

(11) **EP 1 159 969 A1**
(43) Date de publication de la demande: **05.12.2001**
(21) Numéro de dépôt: 00420112.5
(22) Date de dépôt: 30.05.2000
(51) Int. Cl.: A61K 39/295, C12N 7/00

(54) **Vaccine composition**

(71) Demandeur: Aventis Pasteur, 69007 Lyon (FR)
(72) Inventeur: Lang, Jean, 69780 Mions (FR); Saluzzo, Jean-François, 69005 Lyon (FR)
(74) Mandataire: Kerneis, Danièle

(57) **Abrégé**

L'invention a pour objet une composition vaccinale tétravalente contre la Dengue, comprenant des virus de chacune des souches atténuées déposées auprès de la CNCM à l'Institut Pasteur de Paris sous les références I-2480, I-2481, I-2482 et I-2483, caractérisée en ce que la quantité relative de virus de chacun des sérotypes est telle que la quantité de virus du sérotype 3 est inférieure à la quantité de virus des autres sérotypes. En particulier, l'invention a pour objet une composition tétravalente dans laquelle la quantité de virus de chacun des sérotypes 1 à 4 , par dose vaccinante, répond à l'une des formulations suivantes : 3,2,1,2 ou 3,3,1,3 dans lesquelles les chiffres expriment les valeurs des logs10 de la dilution de virus nécessaire pour infecter 50% des cellules.

## Description

L'invention est relative au domaine des vaccins, et en particulier des vaccins vivants atténués. Plus précisément, l'invention est relative à une composition vaccinale tétravalente contre la Dengue dans laquelle la quantité de virus de la Dengue de sérotype 3 est diminuée par rapport à la quantité de virus des autres sérotypes.

La Dengue, ainsi que sa forme sévère, la fièvre hémorragique est une maladie tropicale d'origine virale. Le virus qui en est responsable fait partie de la famille des Flaviridae (genre Flavivirus), qui comprend également le virus de la fièvre jaune ainsi que celui de l'encéphalite japonaise. On connaît 4 sérotypes susceptibles de provoquer la Dengue. Alors que cette maladie est actuellement en progression dans le monde, aucun vaccin n'est actuellement disponible commercialement. En effet, pour pouvoir être proposé avec toutes chances de prévenir efficacement la Dengue, un vaccin doit, tout en présentant des garanties d'inocuité, être capable d'induire chez l'individu auquel il est administré une réponse du système immunitaire susceptible de le protéger lors d'une contamination ultérieure par le virus sauvage. De préférence ce vaccin doit être efficace quelque soit le sérotype du virus sauvage. Or, jusqu'à présent, aucun des candidats-vaccins étudiés n'a présenté toutes les qualités nécessaires.

Un vaccin tétravalent contenant les 4 souches déposées auprès de la CNCM de l'Institut Pasteur de Paris sous les références I-2480, I-2481, I-2482, et I-2483, qui sont des souches atténuées des sérotypes 1, 2, 3 et 4 de la Dengue a déjà été testé sur des êtres humains, ainsi que cela a été décrit par Natth BHAMARAPRAVATI et Sutee YOKSAN au Chapitre 17, pages 373-374 de l'ouvrage *DENGUE AND DENGUE HEMORRHAGIC FEVER* édité par D.J. Gubler et G.Kuno, CAB INTERNATIONAL, 1997. Bien que selon cet art antérieur, tous les sujets de l'essai clinique aient globalement séroconverti, sans souffrir de réactions secondaires autres que légères, on a souhaité améliorer la formulation de la composition vaccinale afin de l'optimiser. On a, en effet, voulu disposer d'une composition vaccinale pouvant être administrée à des êtres humains en toute sécurité tout en induisant une séroconversion vis-à-vis de chacun des sérotypes d'un maximum de sujets vaccinés; la composition vaccinale devant en outre pouvoir être préparée dans des conditions industrielles au moindre coût afin de rendre la vaccination accessible à tous, et notamment aux pays les plus pauvres qui sont parmi ceux les plus touchés par cette maladie.

Pour atteindre ce but, l'invention propose une composition vaccinale tétravalente contre la Dengue, comprenant des virus de chacune des souches atténuées déposées auprès de la CNCM à l'Institut Pasteur de Paris sous les références I-2480, I-2481, I-2482 et I-2483, caractérisée en ce que la quantité relative de virus de chacun des sérotypes est telle que la quantité de virus du sérotype 3 est inférieure à la quantité de virus des autres sérotypes.

Selon une caractéristique de l'invention, la quantité de virus, par dose vaccinante, du sérotype 3 est inférieure d'au moins 1 unité à la quantité de virus des autres sérotypes lorsque le titre viral est exprimé en log10 de la dilution de virus nécessaire pour infecter 50% des cellules.

Selon un mode de réalisation de l'invention, les virus des sérotypes autres que le sérotype 3 peuvent être présents en quantités égales, ou en proportions telles que la quantité de virus, par dose vaccinante, du sérotype 1 est supérieure d'au moins 1 unité à la quantité de virus des autres sérotypes lorsque le titre viral est exprimé en log10 de la dilution de virus nécessaire pour infecter 50% des cellules.

De façon surprenante, il a été remarqué qu'avec de telles formulations, les réponses immunitaires induites chez les personnes vaccinées étaient tout à fait satisfaisantes, alors que les réactions secondaires étaient très fortement diminuées.

Selon un mode particulier de réalisation de l'invention, la quantité de virus de chacun des sérotypes 1 à 4 , par dose vaccinante, répond à l'une des formulations suivantes : 3,2,1,2 ou 3,3,1,3 dans lesquelles les chiffres expriment les valeurs des logs10 de la dilution de virus nécessaire pour infecter 50% des cellules.

Selon une caractéristique de l'invention, la composition vaccinale est destinée à être administrée par injection sous-cutanée.

Selon un mode particulièrement avantageux, l'administration de la composition vaccinale est effectuée en 2 doses séparées d'au moins 1 mois. En effet, on a remarqué qu'on a alors une réponse en anticorps accrûe, et de façon tout à fait inattendue, une fréquence moins grande des réactions secondaires.

On a, en outre remarqué, que l'administration d'une seconde dose permettait de "complémenter" la réponse induite lors de la première dose, en ce sens que les individus n'ayant pas séroconverti vis-à-vis d'un certain sérotype lors de la 1^{ère} dose ont la capacité à le faire lors de la 2^{nde} dose; cette observation, associée à la présence d'une réponse immunitaire de type cellulaire mise en évidence lors d'essais cliniques précédents avec des compositions vaccinales comprenant les mêmes souches vivantes atténuées que celles de la présente invention, conduit à penser que la composition vaccinale proposée par la présente invention permet effectivement de prévenir la maladie de la Dengue.

La présente invention a également pour objet l'utilisation d'une composition virale comprenant des virus de chacune des souches atténuées déposées auprès de la CNCM à l'Institut Pasteur de Paris sous les références I-2480, I-2481, I-2482 et I-2483, dans laquelle la quantité relative de virus de chacun des sérotypes est telle que la quantité de virus du sérotype 3 est inférieure à la quantité de virus des autres sérotypes, pour la fabrication d'une composition vaccinale contre la Dengue.

L'invention a également pour objet une méthode de vaccination contre la Dengue, selon laquelle on administre à un être humain, une composition vaccinale tétravalente contre la Dengue, comprenant des virus de chacune des souches atténuées déposées auprès de la CNCM à l'Institut Pasteur de Paris sous les références I-2480, I-2481, I-2482 et I-2483, caractérisée en ce que la quantité relative de virus de chacun des sérotypes est telle que la quantité de virus du sérotype 3 est inférieure à la quantité de virus des autres sérotypes.

Les souches de virus selon la présente invention ont toutes été obtenues à partir de sérum de patients ayant été atteints de la Dengue en Asie du Sud-Est. Les isolements ont été passés sur cellules de mammifères puis inoculés par voie intra-thoracique (IT) à des moustiques d'élevage (*Toxorhynchites* ou *Aedes aegypti*). Cette étape a permis d'éliminer d'éventuels virus contaminants qui ne seraient pas des arbovirus ; en effet, seuls les arbovirus migrent au niveau des glandes salivaires du moustique après une administration intra-thoracique ; il fallait donc séparer la tête du reste du corps du moustique et la broyer pour obtenir la souche virale purifiée.
Ensuite, les virus des sérotypes 1, 2 et 4 ont été passés sur des cellules primaires de rein de chien (PDK), à 32° C, tous les 5 à 10 jours, alors que le virus du sérotype 3 a été atténué par passage sur des cellules de rein de singe (PGMK) puis cultivé sur des cellules pulmonaires de foetus Rhesus (FRhL).
Les souches atténuées que l'on obtient ont alors les caractéristiques suivantes:
- Lorsqu'on effectue des titrages sur cellules de mammifères, par exemple sur cellules LLC-MK2 qui sont des cellules issues de rein de singe, les souches atténuées donnent de petites plages comparativement aux souches parentales dont elles dérivent.
- Chez le souriceau nouveau-né, lorsqu'elles sont inoculées par voie intra-cérébrale, elles ont une faible virulence.
- Chez le singe, lorsqu'elles sont inoculées par voie sous-cutanée, elles provoquent une virémie faible (voire non-détectable).
- Elles ont une sensibilité à une température proche de 40°C.

Aux termes de la présente invention, on entend par souche dérivée des souches faisant l'objet des dépôts à la CNCM I-2480, I-2481, I-2482 et I-2483, des souches virales obtenues à partir de ces souches, mais qui ont subi des passages supplémentaires sur cellules permissives aux virus, tout en conservant les propriétés mentionnées ci-dessus.

La souche 16007, de sérotype 1, a été isolée en 1964 d'un patient atteint de Dengue associé à un syndrome de choc en Thailande. Le sérum virémique original a été passé sur des cultures de tissu avant d'être inoculé au moustique *Toxorhynchites amboinensis.* Ensuite, le virus obtenu a été passé sur cellules PGMK pour préparer la semence virale-mère. La semence est ensuite passée sur cellules PDK, jusqu'à 43 fois. La souche obtenue après 13 passages sur cellules PDK présente toutes les caractéristiques d'atténuation pour une utilisation vaccinale ; c'est celle faisant l'objet du dépôt I-2480 effectué auprès de la CNCM de l'Institut Pasteur à Paris.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PDK, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2480 est considérée comme faisant partie de la présente invention.

La souche 16681, de sérotype 2, a été isolée d'un patient représentant un cas fatal de Dengue en Thailande en 1964. Cette souche a été passée 2 fois dans des moustiques *Toxorhynchites amboinensis* et une fois sur cellules de rein de singe pour produire la semence-mère. La souche 16681 PDK 53 convenant aux fins de l'invention et objet du dépôt I-2481 effectué auprès de la CNCM a été obtenue après 53 passages successifs hebdomadaires sur des cellules PDK.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PDK, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2481 est considérée comme faisant partie de la présente invention.

La souche 16562, de sérotype 3, a été isolée d'un patient atteint de Dengue à Manille en 1972. Elle a atténuée par 30 passages successifs sur cellules PGMK, puis cultivée sur cellules FRhL. La souche vaccinale 16562 PGMK 30 FRhL 3, objet du dépôt I-2482 effectué auprès de la CNCM et convenant aux fins de l'invention a subi 3 passages successifs sur cellules FRhL après les 30 passages sur cellules PGMK.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PGMK ou FRhL, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2482 est considérée comme faisant partie de la présente invention.

La souche 1036, de sérotype 4, a été isolée d'un enfant atteint de Dengue classique en Indonésie. Cette souche a été atténuée par passages successifs sur des cellules PDK. La souche 1036 PDK 48, convenant aux fins de l'invention et objet du dépôt I-2483 effectué auprès de la CNCM a été passé 48 fois sur cellules PDK.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PDK, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2483 est considérée comme faisant partie de la présente invention.

Selon l'invention, la quantité de virus de chacun des sérotypes est de préférence effectuée selon la méthode décrite dans la demande de brevet WO98/45709 selon laquelle on propage le virus sur des cellules qui sont à la fois permissives au virus mais qui n'induisent pas d'interférence entre les différents sérotypes, telles que les cellules VERO, puis on détermine la quantité de virus de chacun des sérotypes grâce à un anticorps monoclonal spécifique du sérotype que l'on souhaite titrer. Le titre viral est déterminé par la méthode Spearman et Karber et exprimé en dose infectant 50% des cultures cellulaires (CCID₅₀).
Toute autre méthode équivalente à cette méthode, et notamment la méthode des plages dont les résultats sont exprimés en pfu, peut également être utilisée.
De même toute méthode (telle que l'inoculation aux moustiques vivants) dont les résultats seraient exprimés en une unité complètement différente, mais pour laquelle il serait possible de faire la correspondance avec la méthode décrite dans la demande WO98/45709 peut également être utilisée.

Selon l'invention, on entend par dose vaccinante la quantité de composition vaccinale administrée en une fois à une personne que l'on souhaite vacciner. Cette quantité peut notamment être de 0,5ml lors d'une injection sous-cutanée.

Les compositions vaccinales selon l'invention peuvent comprendre tous les éléments habituellement présents dans un vaccin, et notamment stabilisants, conservateurs, adjuvants. Cependant, de bons résultats ont été obtenus sans adjuvant vaccinal.

L'exemple qui suit illustre un mode particulier de réalisation de la présente invention.

On prépare, à partir de suspensions virales vrac comprenant les souches 16007 PDK13, 16681 PDK53, 16562 PGMK30FrhL3 et 1036 PDK48, des compositions tétravalentes ayant les formulations suivantes, dans lesquelles les titres de chacun des sérotypes sont exprimés en log₁₀ de la dilution de virus nécessaire pour infecter 50% des cellules (i.e. en CCID₅₀/dose).

| Groupe | DENGUE 1 | DENGUE 2 | DENGUE 3 | DENGUE 4 |
|---|---|---|---|---|
| A | 4 | 4 | 4 | 4 |
| B | 3 | 2 | 1 | 2 |
| C | 4 | 4 | 1 | 4 |

Les compositions vaccinales sont préparées sous forme de doses de 0,5 ml.
Les sujets de l'essai sont des adultes Thailandais dont l'âge est compris entre 18 et 40 ans.
Le nombre de sujets pour le groupe A est n=11, pour le groupe B n=5, pour le groupe C n=6.
L'administration de la composition vaccinale est faite par injection sous-cutanée dans la région deltoïde, en quantité de 0,5ml pour la 1^{ère} dose et en quantité de 0,5ml pour la 2^{nde} dose qui est administrée 6 mois plus tard.

On effectue des prélèvements sanguins pour réaliser des dosages en anticorps neutralisants, des détections de virémie ainsi que des dosages de différents paramètres biologiques.

D'autre part, tous les symptômes relatifs aux réactions indésirables lors de la vaccination ont été observés et sont rapportés dans le tableau 1 ci-dessous sous forme d'un index de tolérance exprimé en termes de moyenne +/- écart type avec entre parenthèses les valeurs minimale et maximale, que ce soit après la 1^{ère} dose ou après la 2^{nde}.
L'index de tolérance est calculé à partir des réactions systémiques de chaque sujet. Le produit de la durée par la sévérité est calculé pour les réactions systémiques suivantes : fièvre, frissons, malaise, céphalées, myalgie, arthralgie, rash, prurit, douleur occulaire, photophobie et conjonctivite. Puis pour chaque sujet, la valeur maximale entre fièvre et frissons, myalgie et arthralgie, rash et prurit, douleur occulaire, photophobie et conjonctivite est retenue et sommée avec la valeur pour malaise et céphalées. Finalement la moyenne des valeurs des sujets (+/- écart type) est calculé pour chaque groupe. Cet index permet donc, pour chaque sujet, d'évaluer la tolérance en tenant compte du nombre de réactions, de leur sévérité et de leur durée.

**Tableau 1:**

| Index de tolérance | | | |
|---|---|---|---|
| | Groupe A | Groupe B | Groupe C |
| 1^{ère} Dose | 27,4 +/- 18,7 (3-63) | 11 +/- 4,06 (4-14) | 9,2 +/- 9,2 (0-24) |
| 2^{nde} Dose | 2,36 +/- 2,06 (0-6) | 0,6 +/- 0,89 (0-2) | 0,5 +/- 0,55 (0-1) |

Ces résultats montrent clairement que la composition vaccinale selon l'invention (Groupes B et C) conduit à un index plus faible que la composition vaccinale dans laquelle tous les sérotypes sont présents en quantité égale.
On voit également que les réactions indésirables sont bien moins importantes, et même pratiquement inexistantes, lors de la 2^{nde} dose.
Les résultats relatifs aux anticorps neutralisants vis-à-vis de chacun des sérotypes administrés, exprimés en GMT, sont représentés dans les tableaux 2 et 3 ci-dessous.
Les résultats ont été obtenus par test de réduction des plages selon la méthode décrite par Russel et al, "*A plaque réduction test for dengue virus neutralizing antibodies*", Journal of Immunology, 1967;99:285-290, et sont exprimés en inverse de la dilution qui conduit à une réduction de 50% de la plage de lyse sur des cellules LLC-MK2.
Cette méthode consiste à mettre en présence des dilutions de sérums avec un nombre déterminé de particules virales qui inoculées sur les cellules permissive LLCMK2 donnent des plages de lyse, c'est à dire une destruction partielle et localisée du tapis cellulaire. Lorsqu'on réalise le test dans des conditions standardisées la présence d'anticorps neutralisants aboutit à la réduction du nombre des plages de lyse. On exprime le résultat comme la dernière dilution du sérum donnant encore 50% de réduction du nombre total de plages, apprécié comparativement à un test réalisé avec un sérum de référence négatif.

**Tableau 2:**

| Résultats des titres en anticorps neutralisants (moyenne géométrique) vis-à-vis des sérotypes 1 et 2. | | | | | | | | | | |
|---|---|---|---|---|---|---|---|---|---|---|
| | DENGUE 1 | | | | | DENGUE 2 | | | | |
| | 1^{ère} Dose | | | 2^{nde} Dose | | 1^{ère} Dose | | | 2^{nde} Dose | |
| | J0 | J28 | J60 | J0 | J28 | J0 | J28 | J60 | J0 | J28 |
| Groupe A | 5 | 45.3 | 43.3 | 19.9 | 137 | 5 | 39.5 | 35.1 | 25.2 | 189 |
| Groupe B | 5 | 30.2 | 21.6 | 13.5 | 30.8 | 5 | 55.3 | 54.4 | 26 | 91.9 |
| Groupe C | 5 | 5.0 | 8.13 | 15.6 | 60.3 | 5 | 48.6 | 103 | 112 | 353 |
| Un titre de 5 correspond à un résultat négatif. | | | | | | | | | | |

**Tableau 3:**

| Résultats des titres en anticorps neutralisants (moyenne géométrique) vis-à-vis des sérotypes 3 et 4. | | | | | | | | | | |
|---|---|---|---|---|---|---|---|---|---|---|
| | DENGUE 3 | | | | | DENGUE 4 | | | | |
| | 1^{ère} Dose | | | 2^{nde} Dose | | 1^{ère} Dose | | | 2^{nde} Dose | |
| | J0 | J28 | J60 | J0 | J28 | J0 | J28 | J60 | J0 | J28 |
| Groupe A | 5 | 489 | 338 | 240 | 324 | 5 | 22.7 | 24.2 | 18.2 | 101 |
| Groupe B | 5 | 823 | 780 | 109 | 264 | 5 | 27.6 | 15.3 | 6.98 | 15.2 |
| Groupe C | 5 | 224 | 206 | 101 | 205 | 5 | 5 | 5 | 5 | 55 |

Ces résultats montrent que, avec les compositions vaccinales selon la présente invention, la réponse immunitaire en anticorps neutralisants est satisfaisante avec un effet de complémentation de cette réponse immunitaire avec la 2^{nde} dose.

Si l'on exprime ces résultats en taux de séroconversion, i.e. en pourcentage de sujets ayant un titre en anticorps neutralisants supérieur ou égal à 10, on remarque que tous les sujets ont séroconverti vis-à-vis de 3 sérotypes, et que 75% des sujets ayant reçu une composition vaccinale selon l'invention ont séroconverti vis-à-vis des 4 sérotypes.

Ainsi, il est possible, grâce à la présente invention de disposer de compositions vaccinales contre la Dengue ayant un profil de sécurité amélioré mais à caractère immunogène conservé.

## Revendications

1. Composition vaccinale tétravalente contre la Dengue, comprenant des virus de chacune des souches atténuées déposées auprès de la CNCM à l'Institut Pasteur de Paris sous les références I-2480, I-2481, I-2482 et I-2483, **caractérisée en ce que** la quantité relative de virus de chacun des sérotypes est telle que la quantité de virus du sérotype 3 est inférieure à la quantité de virus des autres sérotypes.

2. Composition vaccinale selon la revendication 1, dans laquelle la quantité de virus, par dose vaccinante, du sérotype 3 est inférieure d'au moins 1 unité à la quantité de virus des autres sérotypes lorsque le titre viral est exprimé en log10 de la dilution de virus nécessaire pour infecter 50% des cellules.

3. Composition vaccinale selon une des revendications précédentes, dans laquelle la quantité de virus, par dose vaccinante, du sérotype 1 est supérieure d'au moins 1 unité à la quantité de virus des autres sérotypes lorsque le titre viral est exprimé en log10 de la dilution de virus nécessaire pour infecter 50% des cellules.

4. Composition vaccinale selon une des revendications précédentes selon laquelle la quantité de virus de chacun des sérotypes 1 à 4 , par dose vaccinante, répond à l'une des formulations suivantes : 3,2,1,2 ou 3,3,1,3 dans lesquelles les chiffres expriment les valeurs des logs10 de la dilution de virus nécessaire pour infecter 50% des cellules.

5. Composition vaccinale selon une des revendications précédentes, **caractérisée en ce qu'**elle est destinée à être administrée 2 fois séparées d'un intervalle de temps d'au moins 1 mois.

6. Composition vaccinale selon une des revendications précédentes, **caractérisée en ce qu'**elle est destinée à une administration sous-cutanée.

7. Utilisation d'une composition virale comprenant des virus de chacune des souches atténuées déposées auprès de la CNCM à l'Institut Pasteur de Paris sous les références I-2480, I-2481, I-2482 et I-2483, dans laquelle la quantité relative de virus de chacun des sérotypes est telle que la quantité de virus du sérotype 3 est inférieure à la quantité de virus des autres sérotypes, pour la fabrication d'une composition vaccinale contre la Dengue.
